# FASCICULE DE BREVET EUROPEEN

(11) **EP 4 531 721 B1**
(45) Date de publication et mention de la délivrance du brevet: **27.05.2026**
(21) Numéro de dépôt: 23755019.9
(22) Date de dépôt: 30.05.2023
(51) Int. Cl.: A61B 17/70

(54) **DISPOSITIF DE STABILISATION VERTEBRALE**
WIRBELSTABILISIERUNGSVORRICHTUNG
VERTEBRAL STABILIZATION DEVICE

(30) Priorité: 01.06.2022 FR 2205293
(43) Date de publication de la demande: 09.04.2025
(73) Titulaire: Fayada, Paul, 31410 Mauzac (FR)
(72) Inventeur: Fayada, Paul, 31410 Mauzac (FR)
(74) Mandataire: Cabinet Boettcher
(86) Numéro de dépôt international: PCT/EP2023/064459
(87) Numéro de publication internationale: WO 2023/232824

(56) Documents cités:
- EP-A1- 1 665 994
- WO-A1-98/55038
- DE-A1- 19 950 075
- FR-A1- 2 865 377
- US-A1- 2010 211 100

## Description

La présente invention concerne le domaine des implants chirurgicaux et plus particulièrement les dispositifs de stabilisation vertébrale implantée dans un corps humain ou animal pour le traitement des pathologies du rachis.

Il existe de nombreux dispositifs de stabilisation vertébrale dont le principe général est de relier des vertèbres adjacentes par des tiges.

Dans un premier dispositif, les tiges (parfois appelées barres) sont fixées de part et d'autre des vertèbres à relier selon un plan sensiblement parallèle au plan sagittal médian de la colonne vertébrale. Ce dispositif permet d'obtenir une très bonne stabilisation des vertèbres les unes par rapport aux autres mais a comme principal inconvénient de limiter de manière importante la mobilité de la colonne, gênant voire empêchant certains mouvements du patient. Les tiges sont directement insérées dans les têtes de vis pédiculaires, ce qui impose que les têtes de vis soient parfaitement alignées les unes par rapport aux autres, ce qui est parfois difficile à obtenir.

Dans un second dispositif faisant l'objet de la demande de brevet WO 2018/019792, les tiges sont fixées de telle manière que les tiges se croisent sur le plan sagittal médian de la colonne vertébrale. Ce dernier dispositif permet de conserver une certaine mobilité entre les deux vertèbres reliées par le dispositif tout en renforçant leur stabilité. Les tiges ont une première extrémité en forme d'anneau circulaire et une seconde extrémité en forme d'anneau oblong. Lors de la mise en place, les anneaux sont engagés respectivement sur des tronçons filetés prolongeant les vis pédiculaires vissées directement dans les vertèbres. La fixation définitive des vertèbres se fait en vissant un écrou sur chaque tronçon fileté pour bloquer par serrage l'anneau de la tige, fixant ainsi la position de la tige par rapport aux vis pédiculaires. Ce principe de fixation bien que simple est assez délicat à ajuster précisément. En outre, lors du serrage des écrous, il peut y avoir un léger déplacement de la tige autour des éléments de fixation modifiant l'ajustement initial réalisé par le chirurgien. Ainsi, la mise en place de ce dispositif exige une grande maîtrise et beaucoup de méticulosité de la part du chirurgien, ce qui a, en général, pour conséquence d'allonger la durée des interventions.

La demande FR 2 865 377 A1 divulgue un dispositif de stabilisation vertébrale comprenant une vis en deux parties distinctes : un pion proximal fileté et un corps de vis distal fileté, la tête du pion étant engagée dans une cavité proximale du corps, permettant un débattement multidirectionnel entre le pion et le corps de vis.

### OBJET DE L'INVENTION

L'invention a notamment pour but de pallier au moins en partie les inconvénients précités en proposant un dispositif de stabilisation vertébrale qui soit compact et dont l'implantation soit facile et rapide.

### RESUME DE L'INVENTION

La présente invention est définie dans la revendication 1, tandis que les modes de réalisation préférés sont décrits dans les revendications dépendantes.

A cet effet, on prévoit un dispositif de stabilisation vertébrale comprenant au moins deux vis ayant chacune un premier tronçon fileté pour être engagé dans une vertèbre et un deuxième tronçon fileté séparé du premier tronçon fileté par une collerette. Le dispositif de l'invention comprend en outre au moins une première tige de liaison ayant deux extrémités opposées pour relier l'un à l'autre les deuxièmes tronçons filetés des vis et au moins un premier ensemble de fixation et un deuxième ensemble de fixation pour fixer les extrémités de la tige aux vis. Chaque ensemble de fixation du dispositif objet de l'invention comprend au moins une pince, engageable sur le deuxième tronçon fileté d'une des vis, ladite pince étant agencée pour serrer une extrémité de la tige, et comprend un écrou qui a une surface inférieure d'appui, ledit écrou est agencé pour être vissé sur le deuxième tronçon fileté en serrant la pince directement ou indirectement contre une surface supérieure de la collerette de la vis. Chaque ensemble de fixation est agencé pour créer au moins une liaison rotule entre la pince et le deuxième tronçon fileté.

Ainsi, selon l'invention, le chirurgien pourra réaliser la stabilisation d'au moins deux vertèbres en utilisant au moins un dispositif de stabilisation selon l'invention. La stabilisation des vertèbres est facilement réalisée par l'intermédiaire de tiges simplement fixées par les pinces des ensembles de fixation, indifféremment de façon croisée sur un plan sagittal médian de la colonne vertébrale ou selon un montage parallèle classique dans un plan parallèle au plan sagittal médian de la colonne. Le dispositif selon l'invention permet non seulement une fixation des tiges plus aisée et plus fiable mais également des reprises de montages chez des patients opérés, avec par exemple une extension du montage, sans avoir à démonter l'ensemble comme c'est nécessairement le cas avec les instrumentations disponibles actuellement.

De manière préférée, la surface inférieure d'appui de l'écrou et la surface supérieure d'appui de la collerette sont des surfaces concaves en calotte sphérique identiques l'une à l'autre.

Selon un premier agencement particulier conforme à l'invention, la pince au moins du premier ensemble de fixation comprend un premier mors ayant une surface extérieure convexe en calotte sphérique pour prendre appui, ici directement, sur la surface supérieure de la collerette et un deuxième mors ayant une surface extérieur convexe en calotte sphérique, le premier ensemble de fixation comprenant une première douille d'appui de l'écrou, ladite première douille ayant une surface frontale inférieure concave complémentaire pour prendre appui contre la surface extérieure convexe en calotte sphérique du deuxième mors. La première douille d'appui peut être distincte de l'écrou. De manière privilégiée, la première douille d'appui comprend une surface frontale supérieure convexe en calotte sphérique complémentaire de la surface inférieure de l'écrou pour prendre appui contre celle-ci.

Selon un deuxième agencement particulier conforme à l'invention, la pince au moins du deuxième ensemble de fixation comprend un premier mors ayant une surface extérieure convexe en calotte sphérique complémentaire pour prendre appui contre la surface inférieure de l'écrou et un troisième mors ayant une surface extérieure concave en calotte sphérique. Le deuxième ensemble de fixation comprend en outre une deuxième douille ayant une surface frontale supérieure complémentaire pour prendre appui sur la surface extérieure concave en calotte sphérique du troisième mors. De manière privilégiée, la deuxième douille a une surface frontale inférieure concave en calotte sphérique pour prendre appui sur la surface supérieure de la collerette. Ici, l'écrou permet de serrer la pince indirectement contre la surface supérieure de la collerette de la vis, en effet selon ce deuxième agencement la deuxième douille est entre la collerette et la pince.

La présence d'une douille dans le premier ou le deuxième agencement permet ainsi d'ajuster la hauteur de l'ensemble de fixation sur le deuxième tronçon filetée de la vis permettant ainsi un positionnement des tiges adaptées à la morphologie du patient et à la pathologie à traiter. En particulier, lorsque la stabilisation vertébrale se fait par croisement des tiges selon l'axe sagittal médian, les douilles permettent d'ajuster la hauteur des tiges l'une par rapport à l'autre sans frottement, ou avec un contact maîtrisé.

Selon un troisième agencement conforme à l'invention, le deuxième ensemble de fixation comprend deux pinces à savoir une pince inférieure et une pince supérieure. La pince inférieure comprend un premier mors ayant une surface extérieure convexe en calotte sphérique complémentaire pour prendre appui directement sur la surface supérieure de la collerette et un deuxième mors ayant une surface extérieure convexe en calotte sphérique. La pince supérieure comprend un premier mors ayant une surface extérieure convexe en calotte sphérique complémentaire pour prendre appui contre la surface inférieure de l'écrou et un troisième mors ayant une surface extérieure concave en calotte sphérique complémentaire de la surface extérieure convexe du deuxième mors de la première pince, la surface extérieure concave du troisième mors de la deuxième pince prenant appui sur la surface extérieure convexe du deuxième mors de la première pince.

Selon l'invention, chaque pince comprend une portion élastiquement déformable reliant les mors entre eux de telle manière que ceux-ci soient mobiles l'un par rapport à l'autre entre une position ouverte et une position fermée.

Selon un mode privilégié de réalisation de l'invention, le premier mors est pourvu d'un perçage pour être engagé dans le deuxième tronçon fileté de la vis. Le perçage comprend un premier tronçon en tronc de cône et un deuxième tronçon cylindrique. Le premier tronçon présente une grande section qui débouche sur la surface extérieure du premier mors et une petite section confondue avec une section cylindrique du deuxième tronçon, le deuxième tronçon débouchant sur la surface interne du premier mors.

Le deuxième mors est également pourvu d'un perçage pour être engagé sur le deuxième tronçon fileté de la vis. Le perçage comprend ici un premier tronçon tronconique et un deuxième tronçon tronconique, le premier tronçon ayant une grande section débouchant sur la surface extérieure du deuxième mors et une petite section confondue avec une petite section du deuxième tronçon. Le deuxième tronçon a une grande section qui débouche sur la surface intérieure du deuxième mors. Le centre commun de rotation des surfaces en contact avec l'écrou et la collerette est disposé au centre des petites sections du premier et deuxième tronçons. De manière imagée, le perçage présente une forme analogue à celle d'un diabolo.

De manière optionnelle la collerette de la vis peut être amovible.

Le perçage particulier des pinces, la forme des surfaces extérieures des pinces, ainsi que celle de la surface extérieure de la collerette, et de la surface inférieure de l'écrou tout comme celle des surfaces frontales supérieure et inférieure des douilles permettent de réaliser des liaison rotule entre les différents éléments du dispositif (collerette, pince(s), douille(s), écrou), ce qui permet au chirurgien d'ajuster de manière extrêmement précise et aisée le positionnement des tiges en fonction de la morphologie du patient et de la pathologie à traiter. Une fois, le positionnement optimal obtenu, ce dernier est bloqué par simple serrage de l'écrou sur chacune des vis.

Selon un mode particulier de réalisation de l'invention, la tige présente sur toute sa longueur une section constante. De manière préférentielle, la tige peut être incurvée. Le mot « tige » désigne ici toute pièce allongée et relativement étroite par rapport à sa longueur, quelle que soit sa section, dont des tiges selon le sens commun de ce mot et des barres.

De manière préférentielle, le deuxième tronçon fileté de chaque vis est pourvu d'une portion terminale agencée pour autoriser une liaison en rotation avec un outil de vissage de la vis dans la vertèbre. Il présente également une amorce de rupture pour pouvoir séparer par rupture la portion terminale du deuxième tronçon filetée.

Ainsi, à la fin de l'intervention, après que les vis ont été vissées dans les vertèbres et que le dispositif selon l'invention aura été mis en place, le chirurgien peut raccourcir chacune des vis au niveau de l'amorce de rupture pour rendre le dispositif plus compact et plus confortable pour le patient.

D'autres caractéristiques et avantages de l'invention ressortiront à la lecture de la description qui suit d'un mode de réalisation particulier et non limitatif de l'invention.

### BREVE DESCRIPTION DES DESSINS

Il sera fait référence aux dessins annexés, parmi lesquels :
[Fig. 1] : vue schématique de trois vertèbres en vue de dessus et en légère perspective, équipées d'un dispositif selon un mode particulier de réalisation de l'invention ;
[Fig. 2] : vue schématique en coupe longitudinale d'une des vis de ce dispositif, pourvue d'un ensemble de fixation selon une première configuration comprenant une pince, une douille et un écrou ;
[Fig. 3] : vue schématique en coupe longitudinale d'une des vis de ce dispositif, pourvue d'un ensemble de fixation selon une deuxième configuration comprenant une douille, une pince, et un écrou ;
[Fig. 4] : vue schématique en perspective d'une vis de ce dispositif, pourvue d'un ensemble de fixation selon une troisième configuration comprenant une première pince, une deuxième pince et un écrou ;
[Fig. 5] : vue schématique de cette vis en coupe longitudinale selon le plan V de la figure 4 ;
[Fig. 6] : vue schématique de cette vis en coupe longitudinale selon le plan VI de la figure 4 d'un ensemble de fixation incliné par liaison rotule par rapport à la vis ;
[Fig. 7] : vue schématique éclatée de cette vis et de l'ensemble de fixation ;
[Fig. 8] : vue schématique éclatée, en coupe longitudinale selon le plan V de la figure 4, de cette vis et de l'ensemble de fixation ;
[Fig. 9] : vue schématique en perspective d'une épingle d'une pince.

### DESCRIPTION DETAILLEE DE L'INVENTION

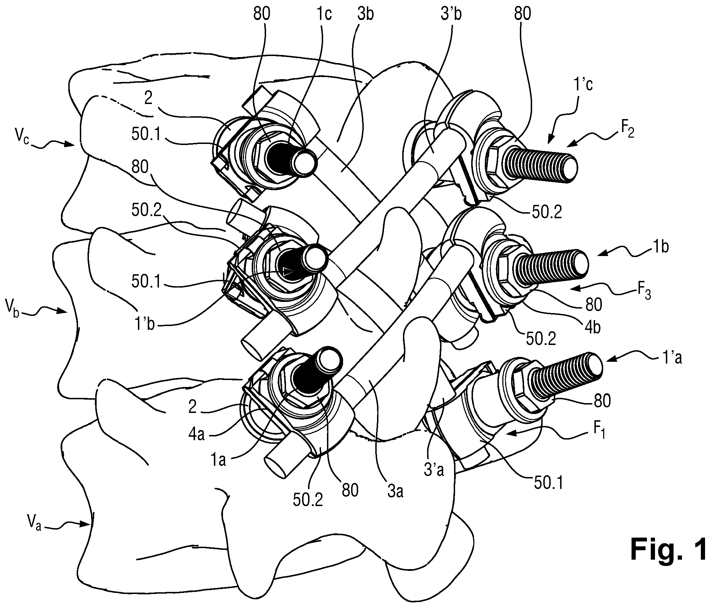

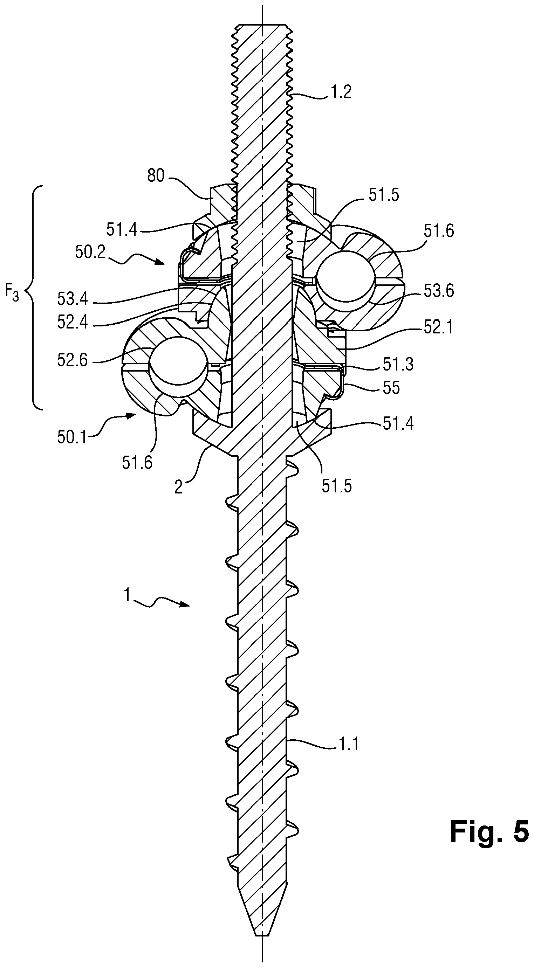

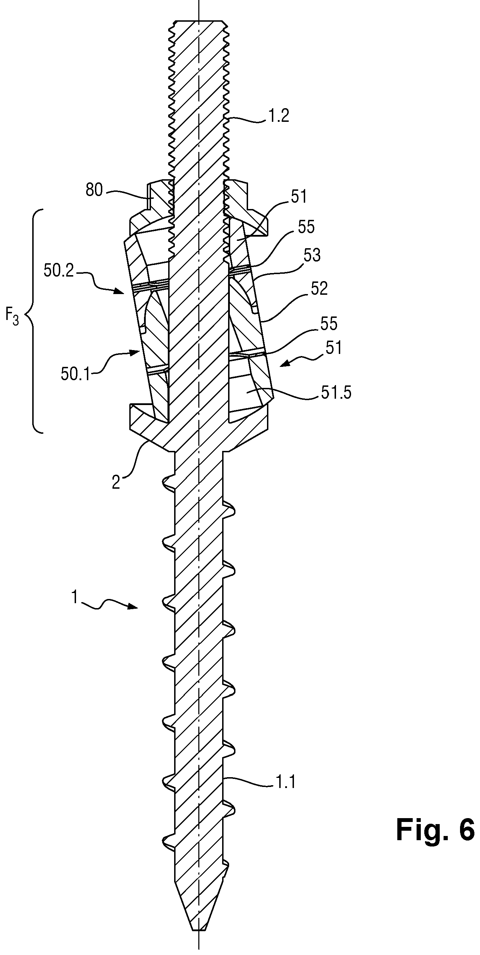

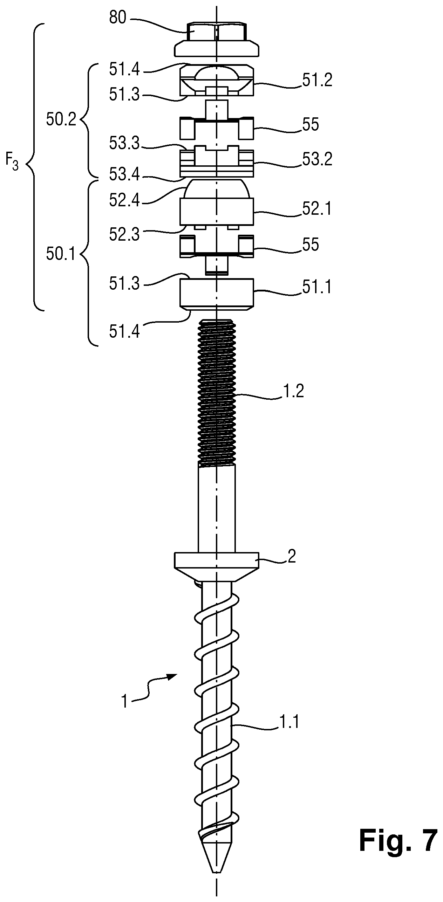

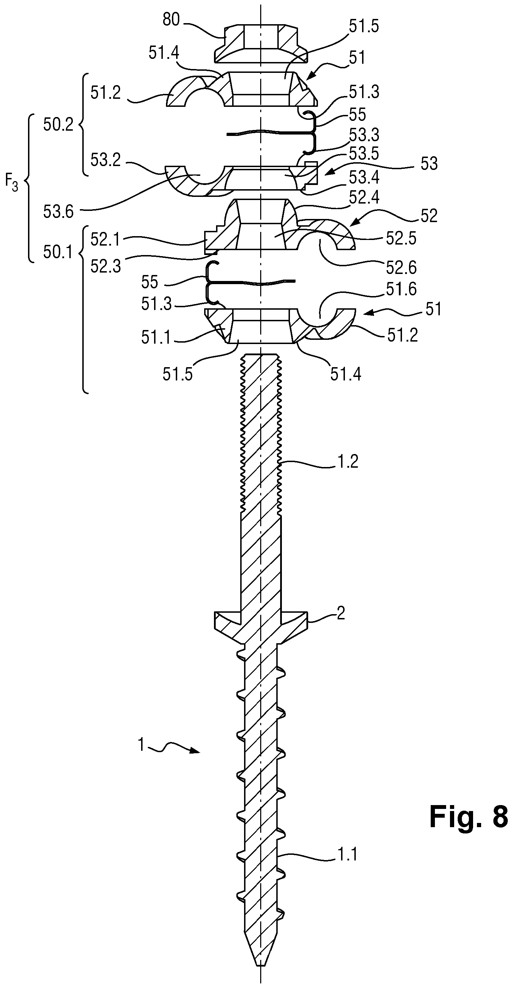

En référence aux figures 1 à 9, la présente invention concerne un dispositif de stabilisation vertébrale comprenant :
- au moins deux vis 1a ; 1b ; chacune munie d'une collerette 2 ;
- au moins une première tige de liaison 3 ;
- au moins un premier ensemble de fixation et un deuxième ensemble de fixation pour fixer les extrémités de la tige 3 respectivement aux vis 1a et 1b. Chaque ensemble de fixation comprend au moins une pince 50.1, 50.2, engageable sur le deuxième tronçon fileté 1.2 d'une des vis et agencée pour serrer une extrémité de la tige 3, et un écrou 80.

Selon un exemple d'appareillage d'une colonne vertébrale représenté à la figure 1, trois vertèbres adjacentes Va, Vb, Vc sont stabilisées avec un dispositif de stabilisation vertébrale conforme à l'invention.

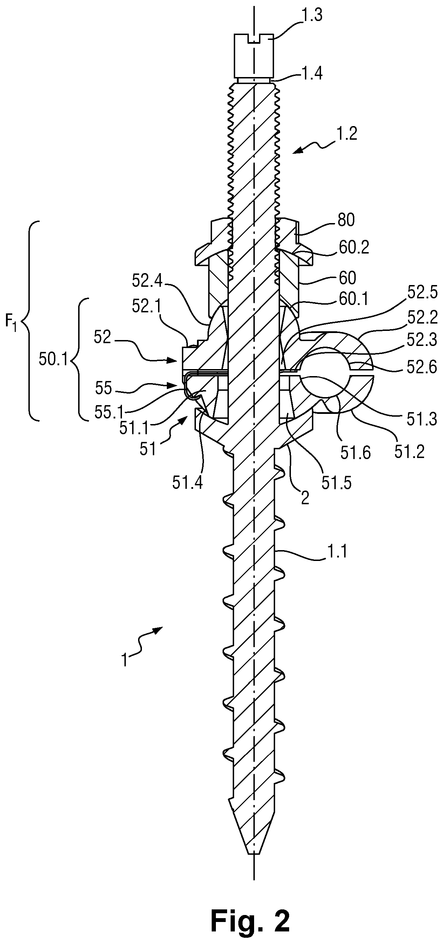

Ici, le dispositif comprend deux vis référencés 1a, 1'a sur la vertèbre Va, deux vis 1b, 1'b sur la vertèbre Vb et deux vis 1c, 1'c sur la vertèbre Vc. Toutes les vis 1, 1' sont identiques,les lettres a, b, c ne servant qu'à distinguer les vis en fonction de la vertèbre sur laquelle elles sont implantées. Chaque vis comporte ainsi un premier tronçon fileté 1.1, non représenté dans la figure 1 mais visible sur les figures 2 à 7, pour être engagé dans la vertèbre et un deuxième tronçon fileté 1.2 séparé du premier tronçon fileté 1.1 par une collerette 2.

Les vertèbres Va, Vb, Vc à instrumenter sont en général préalablement préparées avant de recevoir le dispositif de stabilisation. Classiquement, les apophyses articulaires ou épineuses des vertèbres peuvent avoir été préalablement réduites et les vertèbres préalablement percées pour recevoir les vis.

Comme indiqué précédemment, le premier tronçon fileté 1a.1, 1b.1, 1c.1 des vis 1a, 1b, 1c sont insérés en quinconce dans les vertèbres Va, Vb et Vc de part et d'autre du plan sagittal médian et alternativement. Ainsi, le premier tronçon 1a.1 de la vis 1a est inséré dans la vertèbre Va à gauche du plan sagittal médian, le premier tronçon 1b.1 de la vis 1b est inséré dans la vertèbre Vb à droite du plan sagittal médian et le premier tronçon 1c.1 de la vis 1c est inséré dans la vertèbre Vc à gauche du plan sagittal médian. De manière symétrique, le premier tronçon 1'a.1 de la vis 1'a est inséré dans la vertèbre Va à droite du plan sagittal médian plus ou moins en regard de la vis 1a, De la même façon, le premier tronçon 1'b.1 de la vis 1'b est inséré dans la vertèbre Vb à gauche du plan sagittal médian plus ou moins en regard de la vis 1b et le premier tronçon 1'c.1 de la vis 1'c est inséré dans la vertèbre Vc à droite du plan sagittal médian plus ou moins en regard de la vis 1c. Ainsi, chaque vertèbre Va, Vb, Vc est équipée de deux vis respectivement 1a et 1'a ; 1b et 1'b ; 1c et 1'c.

Toutes les vis étant identiques, la description ci-après d'une vis s'applique à toutes. Comme précédemment indiqué, chaque vis 1 comprend un premier tronçon 1.1 et un deuxième tronçon 1.2, séparés par une collerette 2. Le premier tronçon 1.1 est complètement inséré dans l'os de la vertèbre jusqu'à la collerette 2. La collerette 2 se trouve ainsi à la surface de la vertèbre, en appui sur l'os, le deuxième tronçon de la vis 1.2 étant en saillie de la vertèbre.

Le chirurgien sait percer et positionner correctement les vis 1 en fonction de la morphologie du patient.

L'homme du métier saura conformer le premier tronçon 1.1 de telle sorte à faciliter son engagement dans l'os. Par exemple, le premier tronçon peut être auto-taraudeur et présenter une extrémité taillée en forme de pointe. En variante, le premier tronçon peut être également auto-perceur.

La collerette 2 qui sépare le premier et le deuxième tronçon 1.1 ; 1.2 de la vis 1 présente une surface inférieure convexe en calotte sphérique en contact avec l'os de la vertèbre et une surface supérieure d'appui concave en calotte sphérique. L'homme du métier saura adapter le diamètre et la convexité de la collerette en fonction de l'anatomie du patient et des contraintes mécaniques subies.

Le deuxième tronçon 1.2 de la vis 1 vient ainsi en saillie de la vertèbre lorsque la vis 1 est fixée. Le filetage du deuxième tronçon 1.2 est réalisé de telle sorte à recevoir un ensemble de fixation F. De manière préférentielle, le deuxième tronçon fileté 1.2 est pourvu d'une portion terminale agencée pour autoriser une liaison en rotation avec un outil de vissage. La portion terminale, représentée sur la figure 2 uniquement, peut ainsi comprendre une empreinte 1.3 de forme complémentaire à celle de la pointe d'un tournevis. A titre d'exemples non limitatifs, l'empreinte peut être hexagonale, cruciforme, étoilée ou triangulaire. En outre, le deuxième tronçon fileté 1.2 peut comporter une amorce de rupture 1.4 pour pouvoir séparer par rupture la portion terminale du deuxième tronçon fileté lorsque l'intervention chirurgicale est terminée.

L'homme du métier saura fabriquer la vis, ici monobloc et d'un seul tenant entre le premier tronçon de vis, le deuxième tronçon de vis et la collerette, en matériau biocompatible et inoxydable suffisamment résistant mécaniquement et chimiquement pour l'application envisagée.

L'homme du métier saura déterminer le diamètre et la longueur de la vis pour être les mieux adapter à l'âge, à la morphologie et à la qualité osseuse du patient. A titre d'illustration, la longueur des vis peut être classiquement comprise entre 65mm et 90mm.

Selon ce mode de réalisation particulier de l'invention, le dispositif comprend chacun deux paires de tiges de liaisons, respectivement référencées 3a, 3b et 3'a, 3'b. Chaque tige présente deux extrémités opposées pour relier l'un à l'autre et deux à deux les deuxièmes tronçons filetés de deux des vis.

Ainsi, comme illustré dans la figure 1, une première tige de liaison 3a va relier le deuxième tronçon 1a.2 de la vis 1a au deuxième tronçon 1b.2 de la vis 1b et une deuxième tige de liaison 3b va relier le deuxième tronçon 1b.2 de la vis 1b au deuxième tronçon 1c.2 de la vis 1c de sorte que les vis 1a, 1b, 1c sont reliées deux à deux. De la même manière, une troisième tige 3'a va relier le deuxième tronçon 1'a.2 de la vis 1'a au deuxième tronçon 1'b.2 de la vis 1'b, et une quatrième tige 3'b va relier le deuxième tronçon 1'b.2 de la vis 1'b au deuxième tronçon 1'c.2 de la vis 1'c de sorte que les vis 1'a, 1'b, 1'c sont reliées deux à deux. Les tiges 3a, 3'b sont ainsi parallèles entre elles comme les tiges 3'a et 3b le sont également entre elles. Les tiges 3a et 3'a stabilisent les vertèbres Va et Vb entre elles, les tiges 3b et 3'b les vertèbres Vb et Vc en se croisant sur le plan sagittal médian de la colonne respectivement entre les vertèbres Va et Vb et entre les vertèbres Vb et Vc.

Chaque tige 3 est ici réalisée en matériau biocompatible et présente sur toute sa longueur une section constante. La section est ici circulaire.

Les tiges 3 peuvent être en tout matériau biocompatible suffisamment rigide pour obtenir la stabilisation des vertèbres entre elles. On choisira par exemple un matériau ayant un module de Young compris entre 3,6 GPa et 60 GPa. A titre d'exemple, le matériau peut-être un matériau composite comprenant un mélange de fibres de carbone et de polyétheréthercétone (PEEK) .

L'homme du métier est à même de déterminer le diamètre de la section de la tige en fonction du matériau utilisé, des caractéristiques mécaniques recherchées et de l'anatomie du patient. Il saura également déterminer la longueur des tiges pour l'adapter à la morphologie et à la pathologie du patient et selon que les tiges sont placées parallèlement au plan sagittal médian ou selon qu'elles se croisent sur celui-ci.

De manière préférentielle et selon le mode de réalisation particulier de l'invention, les tiges sont incurvées, facilitant ainsi leur mise en place et limitant les interférences avec la partie centrale des vertèbres accouplées.

La fixation de chaque extrémité des tiges 3 à l'une des vis 1 est assurée par un ensemble de fixation F engagé sur le deuxième tronçon filetée de la vis.

Chaque ensemble de fixation F conforme à l'invention comprend au moins une pince 50.1, 50.2 engageable sur le deuxième tronçon fileté 1.2 d'une des vis 1 et agencée pour serrer une extrémité de la tige 3, et un écrou 80 qui a une surface inférieure d'appui et qui est agencé pour être vissé sur le deuxième tronçon fileté 1.2 en serrant la pince 50.1, 50,2 contre la surface supérieure de la collerette 2 de la vis 1. Chaque ensemble de fixation F est agencé pour créer une liaison rotule entre la pince 50.1, 50.2 et le deuxième tronçon fileté 1.2. De manière préférentielle, la surface inférieure d'appui de l'écrou 80 et la surface supérieure d'appui de la collerette 2 telles que précédemment décrites sont des surfaces concaves en calotte sphérique identiques l'une à l'autre.

L'ensemble de fixation F peut présenter plusieurs configurations selon qu'il se trouve sur une vis implantée dans une des vertèbres d'extrémité de la partie de colonne appareillée (vertèbres Va, Vc) ou sur une vis implantée dans une vertèbre intermédiaire (vertèbre Vb). L'ensemble de fixation F comprendra ainsi une douille dans les deux configurations adaptées aux vertèbres d'extrémité ou une deuxième pince dans la configuration adaptée aux vertèbres intermédiaires.

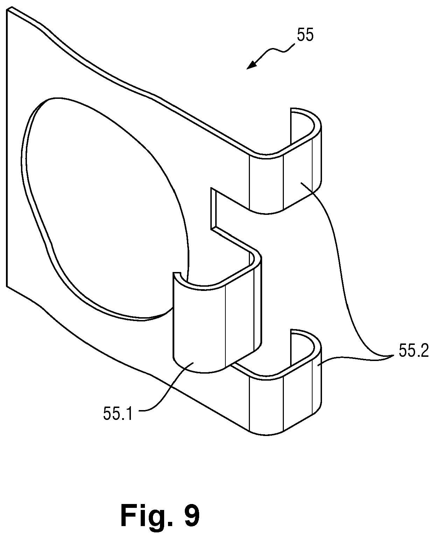

En référence à la figure 2, l'ensemble de fixation F1 selon la première configuration comprend une pince 50.1, une douille 60 et un écrou 80. La pince 50.1 est ici de forme plus ou moins rectangulaire et comprend deux mors 51, 52 reliés par une portion élastiquement déformable, ici une épingle 55 en métal à ressort, de telle manière que les mors soient mobiles l'un par rapport à l'autre entre une position ouverte et une position fermée. L'épingle 55 illustrée à la figure 9 comprend une première patte 55.1 en forme de crochet qui enserre élastiquement une portion de la première partie 51.1 à l'opposé de la deuxième partie 51.2 et deux pattes 55.2 en forme de crochet qui enserrent élastiquement une portion de la première partie 52.1 opposée à la deuxième partie 52.2. L'homme du métier saura réaliser la portion élastiquement déformable de manière différente. Il pourra par exemple réaliser la pince en une seule pièce, les deux mors étant reliées l'un à l'autre par une portion élastiquement déformable issue de matière. Les deux mors peuvent être reliés l'un à l'autre par une articulation éventuellement associée à un ressort en spirale.

Chaque mors 51 comprend une première partie 51.1, ou partie de liaison à la vis 1, destinée à être engagée sur le deuxième tronçon 1.2 de la vis 1, et une deuxième partie 51.2, ou partie de liaison à la tige 3, qui s'étend en saillie latérale par rapport à la première partie 51.1 et qui est agencée pour recevoir une des extrémités d'une des tiges 3.

La première partie 51.1 est ici de forme plus ou moins parallélépipédique avec une section transversale carrée de côté sensiblement égal ou légèrement supérieur au diamètre de la collerette 2. La première partie 51.1 comprend une surface intérieure 51.3 faisant face au mors 52 (et donc tournée vers l'intérieur de la pince 50.1) et à l'opposé une surface extérieure 51.4 tournée vers l'extérieur de la pince 50.1. La surface intérieure 51.3 est une surface plane. La surface extérieure 51.4 est de forme convexe en calotte sphérique complémentaire de celle de la surface supérieure de la collerette 2 de manière à pouvoir prendre un appui surfacique sur celle-ci.

La première partie 51.1 du premier mors 51 est pourvue d'un perçage 51.5 pour être engagé sur le deuxième tronçon fileté 1.2 de la vis 1. Le perçage 51.5 débouche d'un côté sur la surface intérieure 51.3 de la première partie 51.1 du mors perpendiculairement à celle-ci et de l'autre côté sur la surface extérieure 51.4. Le perçage 51.5 comprend ici, le long d'un axe central, un premier tronçon tronconique et un deuxième tronçon cylindrique, le premier tronçon ayant une grande section débouchant sur la surface extérieure 51.4 et une petite section confondue avec une section cylindrique du deuxième tronçon, le deuxième tronçon débouchant sur la surface intérieure 51.3.

La deuxième partie 51.2 comprend une rainure hémicylindrique 51.6 d'axe central perpendiculaire à l'axe central du perçage 51.5 et de rayon sensiblement égal ou légèrement inférieur au rayon de la tige 3.

Le deuxième mors 52 comprend lui aussi une première partie 52.1, ou partie de liaison à la vis 1, destinée à être engagée sur le deuxième tronçon 1.2 de la vis 1 et une deuxième partie 52.2, ou partie de liaison à la tige 3, qui s'étend en saillie latérale par rapport à la première partie et qui est agencée pour recevoir une des extrémités d'une des tiges.

La première partie 52.1 est ici de forme plus ou moins parallélépipédique avec une section transversale carrée identique à celle de la première partie 51.1 et comprend une surface intérieure 52.3 faisant face au mors 51 (et donc tournée vers l'intérieur de la pince 50.1) et à l'opposé une surface extérieure 52.4 tournée vers l'extérieur de la pince 50.1. La surface intérieure 52.3 est une surface plane. La surface extérieure 52.4 de forme convexe en calotte sphérique.

La première partie 52.1 du deuxième mors 52 est également pourvu d'un perçage 52.5 pour être engagé sur le deuxième tronçon fileté 1.2 de la vis 1. Le perçage 52.5 comprend ici un premier tronçon tronconique et un deuxième tronçon tronconique, le premier tronçon ayant une grande section débouchant sur la surface extérieure 52.4 et une petite section confondue avec une petite section du deuxième tronçon, le deuxième tronçon ayant une grande section débouchant sur la surface intérieure 52.3.

La deuxième partie 52.2 est identique à la deuxième partie 51.2 et fait face à celle-ci de telle manière que les rainures 51.6 et 52.6 délimitent un logement cylindrique pour accueillir l'extrémité de la tige 3.

La pince 50.1 est formée en assemblant le premier mors 51 avec le deuxième mors 52 par l'épingle 55. En position fermée, les rainures 51.6, 52.6 des deuxièmes parties 51.2, 52.2 des mors 51 et 52 forment un logement de section transversale circulaire inférieure à celle des extrémités de tige 3 permettant d'y enserrer une extrémité de la tige 3.

L'ensemble de fixation F1 comprend en outre une première douille d'appui 60 de l'écrou 80. La douille est préférentiellement distincte de l'écrou 80 comme cela est représenté dans la figure 2. La première douille d'appui 60 délimite un conduit traversant pour pouvoir être engagée sur le deuxième tronçon 1.2 de la vis 1. Le conduit est ici cylindrique. La douille 60 comprend ici une surface frontale supérieure convexe 60.2 en calotte sphérique complémentaire de la surface inférieure de l'écrou 80 pour prendre appui contre celle-ci et une surface frontale inférieure 60.1 concave en calotte sphérique complémentaire de la surface extérieure 52.4 de la première partie 52.1 du deuxième mors 52.

Ainsi une vis 1 équipée de l'ensemble de fixation F1 selon la première configuration comprend successivement engagés le long du deuxième tronçon 1.2 de vis : une pince 50.1 dont le premier mors 51 est en contact avec la collerette 2, et dont le deuxième mors 52 vient en appui de la surface frontale inférieure 60.1 de la première douille 60, la surface frontale supérieure 60.2 de ladite première douille 60 étant en appui de la surface inférieure de l'écrou 80. Le perçage du premier et du deuxième mors 51, 52 et les surfaces extérieures 51.4, 52.4 coopérant avec la surface supérieure de la collerette 2 et la surface inférieure de la douille 60 permettent de créer une liaison rotule autour de la vis.

On notera que le centre de la calotte sphérique de la surface extérieure 51.4 et le centre de la calotte sphérique de la surface extérieure 52.4 sont confondus et disposés au centre des petites sections des premier et deuxième tronçons du perçage 52.5.

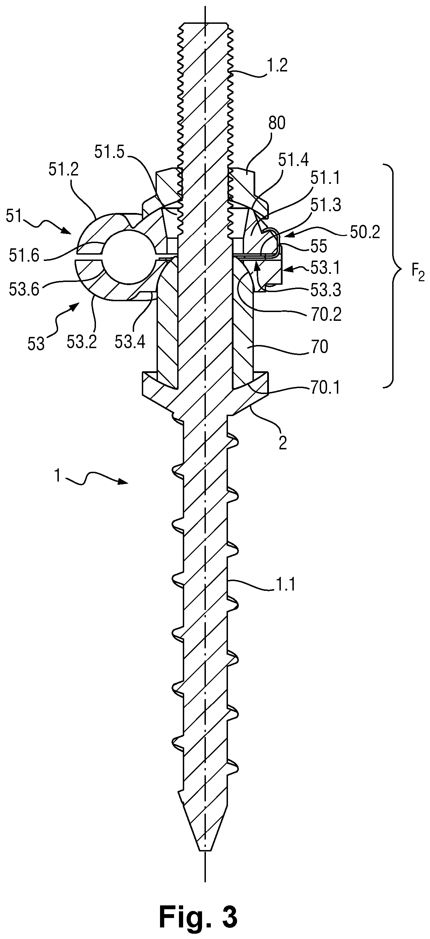

Sur la figure 3, on a représenté un ensemble de fixation F2 selon une deuxième configuration. L'ensemble de fixation F2 comprend une deuxième douille 70, une pince 50.2 et un écrou 80.

La pince 50.2 est composé de deux mors 51, 53 reliés par une épingle élastiquement déformable 55. : un premier mors 51 et un troisième mors 53.

Le premier mors 51 est identique à celui précédemment décrit mais est positionné pour prendre appui contre la surface inférieure de l'écrou 80. On rappelle que la surface inférieure d'appui de l'écrou 80 et la surface supérieure d'appui de la collerette 2 étant de formes identiques, la surface extérieure 51.4 peut prendre appui indifféremment contre l'une ou l'autre en formant une liaison rotule.

Le troisième mors 53 comprend lui aussi une première partie 53.1, ou partie de liaison à la vis 1, destinée à être engagée sur le deuxième tronçon 1.2 de la vis 1 et une deuxième partie 53.2, ou partie de liaison à la tige 3, qui s'étend en saillie latérale par rapport à la première partie 53.1 et qui est agencée pour recevoir une des extrémités d'une des tiges.

La première partie 53.1 est ici de forme plus ou moins parallélépipédique avec une section transversale carrée identique à celle de la première partie 51.1 et comprend une surface intérieure 53.3 faisant face au mors 51 (et donc tournée vers l'intérieur de la pince 50.2) et à l'opposé une surface extérieure 53.4 tournée vers l'extérieur de la pince 50.1. La surface intérieure 53.3 est une surface plane. La surface extérieure 53.4 de forme concave en calotte sphérique.

La première partie 53.1 du troisième mors 53 est également pourvu d'un perçage 53.5 pour être engagé sur le deuxième tronçon fileté 1.2 de la vis 1. Le perçage 53.5 débouche en bas sur la surface extérieure 53.4 et en haut sur la surface intérieure 53.3. Compte tenu de la concavité de la surface extérieure 53.4 qui rentre dans la première partie 53.1, le perçage 53.5 a une longueur minime.

La deuxième partie 53.2 est identique à la deuxième partie 51.2 et fait face à celle-ci de telle manière que les rainures 51.6 et 53.6 délimitent un logement cylindrique pour accueillir l'extrémité de la tige 3.

La deuxième douille 70 délimite un conduit traversant pour pouvoir être engagée sur le deuxième tronçon 1.2 de la vis 1. Le conduit est ici cylindrique. Il peut être fileté ou non. La deuxième douille 70 comprend ici une surface frontale inférieure 70.1 en calotte sphérique convexe complémentaire de la face supérieure de la collerette 2 et une surface frontale supérieure 70.2 en calotte sphérique concave complémentaire de la surface extérieure concave 53.4 de la première partie 53.1 du troisième mors 53.

Ainsi, une vis 1 équipée de l'ensemble de fixation F2 dans la deuxième configuration comprend successivement engagés le long du deuxième tronçon 1.2 de vis 1 : une deuxième douille 70 dont la surface frontale inférieure 70.1 est en appui contre la collerette 2, une pince 50.2 dont le troisième mors 53 a sa surface extérieure 53.4 en appui de la surface frontale supérieure 70.2 de la deuxième douille 70 et dont le premier mors 51 a sa surface extérieure 51.4 en appui contre la surface inférieure d'appui de l'écrou 80. Le perçage 51.5 du premier mors 51 ainsi que les surfaces extérieures 51.4, 53.4 coopérant avec la surface inférieure de l'écrou 80 et la surface frontale supérieure 70.2 de la deuxième douille 70 permettent de créer une liaison rotule de la pince 50.2 autour de la vis 1.

On notera que le centre de la calotte sphérique de la surface extérieure 51.4 et le centre de la calotte sphérique de la surface extérieure 53.4 sont confondus.

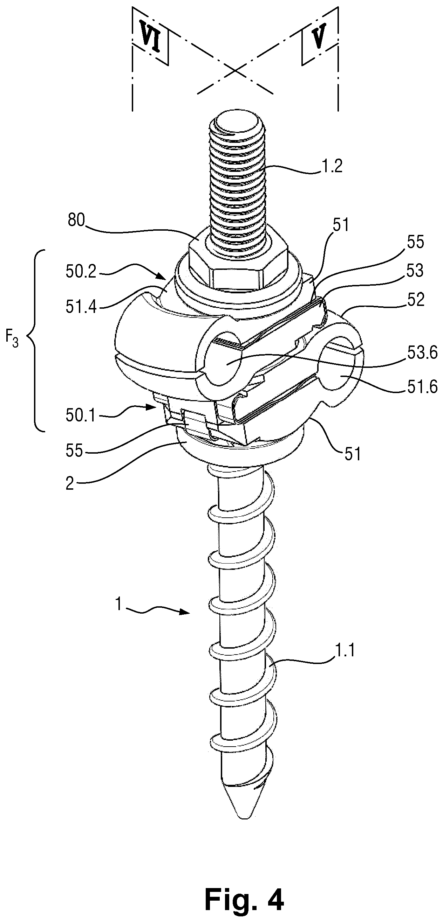

En référence aux figures 4 à 8, un ensemble de fixation F3 selon une troisième configuration comprend deux pinces, une pince inférieure 50.1 et une pince supérieure 50.2. La pince inférieure 50.1 est identique à celle de l'ensemble de fixation F1 précédemment décrit et la pince 50.2 est identique à celle de l'ensemble de fixation F2 précédemment décrit. La surface extérieure 51.4 du mors 51 de la pince inférieure 50.1 vient prendre appui sur la surface supérieure de la collerette 2 ; la surface extérieure 52.4 du mors 52 de la pince inférieure 50.1 prend appui contre la surface extérieure 53.4 du mors 53 de la pince supérieure 50.2 ; la surface extérieure 51.4 du mors 51 de la pince supérieure 50.2 vient prendre appui sur la surface inférieure de l'écrou 80. On assure ainsi une liaison rotule des pinces 50.1 et 50.2 sur la vis 1.

Ainsi, une vis 1 équipée de l'ensemble de fixation F3 selon la troisième configuration comprend successivement, une pince inférieure 50.1 dont le premier mors 51 a sa surface extérieure 51.4 en appui contre la surface supérieure de la collerette 2 et dont le deuxième mors 52 a sa surface extérieure 52.4 en appui contre la surface extérieure 53.4 du troisième mors 53 formant avec un autre premier mors 51 la pince supérieure 50.2, la surface extérieure 51.4 du premier mors 51 de la pince supérieure 50.2 est en appui contre la surface inférieure de l'écrou 80. Le perçage des premiers mors 51 de la première pince inférieure 50.1 et de la deuxième pince supérieure 50.2, le perçage « en diabolo » du deuxième mors 52 de la pince inférieure 50.1, et les surfaces complémentaires en calotte sphérique en contact (la surface extérieure 51.4 de la pince supérieure 50.2 avec la surface inférieure de l'écrou 80 ; la surface extérieure 51.4 de la pince inférieure 50.1 avec la surface supérieure de la collerette 2 ; la surface extérieure 52.4 de la pince inférieure 50.1 avec la surface extérieure 53.4 de la pince supérieure 50.2) permettent de créer une liaison rotule des deux pinces (individuellement et collectivement) autour de la vis 1.

On notera que le centre de la calotte sphérique des deux surfaces extérieures 51.4 sont confondus et disposés au centre des petites sections des premier et deuxième tronçons tronconiques du perçage 52.5.

Ainsi, dans l'implantation illustrée à la figure 1 pour stabiliser trois vertèbres adjacentes Va, Vb, Vc, le chirurgien va utiliser six vis 1, quatre tiges 3 et six ensembles de fixation.

Ainsi, la première vertèbre Va est équipée, à gauche du plan sagittal médian, d'une vis 1a pourvue de l'ensemble de fixation F1 et à droite du plan sagittal médian d'une vis 1'a pourvue de l'ensemble de fixation F2.

La deuxième vertèbre Vb est équipée, à gauche du plan sagittal médian, d'une vis 1'b pourvue de l'ensemble de fixation F3 et à droite du plan sagittal médian d'une vis 1b pourvue de l'ensemble de fixation F3.

La troisième vertèbre Vc est équipée, à gauche du plan sagittal médian, d'une vis 1c pourvue de l'ensemble de fixation F2 et à droite du plan sagittal médian d'une vis 1'c pourvue de l'ensemble de fixation F1.

Une fois les vis insérées dans les vertèbres, le chirurgien va positionner les tiges 3a, 3'a, 3b, 3'b en commençant par celles qui se trouvent au plus près de l'épine dorsale. Ainsi, la tige 3'a a une première extrémité fixée par la pince 50.1 de l'ensemble de fixation F1 de la vis 1'a, la deuxième extrémité de la tige 3'a étant fixée par la pince inférieure 50.1 de l'ensemble de fixation F3 de la vis 1'b. la tige 3b est, elle, fixée à chacune de ses extrémités respectivement par la pince inférieure 50.1 de l'ensemble de fixation F3 de la vis 1b et par la pince 50.1 de l'ensemble de fixation F1 de la vis 1c.

Les tiges 3a et 3'b sont ensuite respectivement fixées. La tige 3a est fixée par une première extrémité à la pince 50.2 de l'ensemble de fixation F2 de la vis 1a et par une deuxième extrémité à la pince supérieure 50.2 de l'ensemble de fixation F3 de la vis 1b. La tige 3'b est quant à elle fixée entre les pinces 50.2 de la vis 1'b et 50.2 de la vis 1'c.

Les tiges 3a et 3'b sont fixées de telle sorte qu'elles passent respectivement au-dessus des tige 3'a et 3b, les tiges se croisant sensiblement sur le plan sagittal médian. Les tiges 3'a et 3a stabilisent ainsi les vertèbres Va et Vb l'une par rapport à l'autre, les tiges 3'b et 3b stabilisent les vertèbres Vb et Vc l'une par rapport à l'autre.

Le chirurgien saura ajuster le positionnement des tiges grâce à la rotation des pinces autour de chaque vis 1 et aux liaisons rotules autour de chacune des vis 1, rendues possibles par les perçages des mors des pinces. Ceci permet donc un ajustement extrêmement précis selon les trois dimensions de l'espace : en hauteur par le positionnement des pinces le long des deuxième tronçons de vis, en longueur par la rotation des pinces autour des vis et en inclinaison grâce aux liaisons rotules, tout en conservant une très grande compacité du dispositif selon l'invention. Une fois les tiges dans leur position optimale, le chirurgien va fixer le dispositif en serrant l'écrou 80 sur chaque vis et couper le deuxième tronçon de chaque vis au niveau de l'amorce de rupture 1.4 (représentée sur la figure 2).

Pour d'autres pathologies, on peut ne stabiliser que deux vertèbres adjacentes. Ici, l'ensemble utilisé comprend deux paires de vis (1a, 1b et 1'a, 1'b). Les vis 1'a, 1'b sont équipées de l'ensemble de fixation F1 tandis que les vis 1a et 1b sont équipées de l'ensemble de fixation F2. Les vis 1a et 1'a sont insérées de part et d'autre de la première vertèbre, respectivement à gauche et à droite du plan sagittal médian, les vis 1b et 1'b sont insérées de part et d'autre de la deuxième vertèbre, respectivement à droite et à gauche du plan sagittal médian. Une première tige 3 est ensuite fixée entre les vis 1'a et 1'b, une deuxième tige 3 entre les vis 1a et 1b.

Ainsi, le dispositif conforme à l'invention utilisé pour stabiliser les vertèbres est facile à mettre en place et peu encombrant. Le nombre de vis de chaque dispositif est établi en fonction du nombre de vertèbres à appareiller. L'utilisation du dispositif selon l'invention permet un réglage optimal et précis de la position des tiges au regard de la pathologie du patient et de sa morphologie. En outre, il est tout à fait possible de modifier facilement ce réglage au fur et à mesure de l'évolution de la pathologie simplement en modifiant la position respective des pinces, en changeant l'ensemble de fixation engagé sur la vis ou en changeant les tiges si celles-ci ne conviennent plus, sans envisager une opération longue pour le patient.

Cette grande précision de réglage du positionnement des tiges, le choix de leur longueur et leur ajustement sur les mors des pinces en fait un système sur mesure adapté à chaque patient, bien que comportant des éléments de fixation en eux-même de fabrication standard.

Pour faciliter encore la mise en place du dispositif, chaque élément de celui-ci (vis, ensembles de fixation : mors, douilles, écrous et tiges) peut faire l'objet d'un marquage ou couleur permettant à la fois d'identifier rapidement l'élément mais aussi de le tracer de la fabrication à la mise en place dans le corps du patient. Le marquage peut être réalisé par tout moyen biocompatible connu en tant que tel.

Bien entendu, l'invention n'est pas limitée aux modes de réalisation décrits mais englobe toute variante entrant dans le champ de l'invention telle que définie par les revendications.

En particulier, les vertèbres instrumentées sont ici adjacentes mais elles pourraient ne pas l'être. Il conviendra alors simplement d'adapter la longueur des tiges en fonction de la distance entre les deux vertèbres à instrumenter.

Les tiges sont ici fixées entre les vertèbres de manière croisée mais le dispositif selon l'invention peut tout à fait permettre la fixation de tiges de manière parallèle au plan sagittal médian. En outre, bien qu'ici un seul mode de fixation soit présenté (fixation avec tiges croisées), on peut prévoir, selon la pathologie du patient, d'utiliser les ensembles de dispositifs selon l'invention pour instrumenter certaines vertèbres de manière croisée et d'autres vertèbres de manière parallèle.

La tige a ici une section circulaire mais elle pourrait avoir une section ovale, triangulaire, rectangulaire, polygonale, etc...

La tige a ici une section constante mais elle pourrait avoir une section plus étroite aux extrémités qu'au centre, ou à l'inverse, plus large aux extrémités qu'au centre.

Les tiges ont ici une longueur prédéfinie mais elles pourraient être coupées à façon à la longueur souhaitée.

Les tiges pourraient être rectilignes au lieu d'être incurvée.

Les vis sont ici pleines mais elles pourraient être creuses pour permettre la fixation ou le passage de broche pour faciliter une chirurgie percutanée.

Les mors ont ici une forme sensiblement parallélépipédique mais peuvent prendre toute forme, par exemple cylindrique, polyédrique, ou ovoïde... La rainure de la deuxième partie peut présenter une section angulaire ou polygonale de sorte qu'une fois les deux mors de la pince fermés, les deuxièmes parties des mors forme un canal de section carrée ou polygonale.

Le dispositif pourrait comprendre des écrous intégrant en une seule pièce des douilles 60, et/ou des vis intégrant en une seule pièce des douilles 70 et/ou des vis et des écrous tels que décrits ici.

Les ensembles de fixations F1 et F2 comprennent ici une douille mais celle-ci pourrait être remplacée par une ou plusieurs rondelles empilables en fonction de la hauteur recherchée, la première et la dernière rondelle reprenant la forme des surfaces frontales inférieure et supérieure de la douille, ou la douille pourrait être complètement absente de l'ensemble de fixation si l'anatomie du patient le permet.

En particulier, ici le dispositif de l'invention s'applique à l'être humain mais il peut être adapté à tout vertébré, en particulier les animaux domestiques comme les équidés ou les chiens. Bien évidemment, l'homme du métier saura adapter les dimensions des éléments du dispositif en fonction, en particulier la longueur et le diamètre des vis et des tiges, et en conséquence les dimensions des pinces et, des douilles et de l'écrou.

## Revendications

1. Dispositif de stabilisation vertébrale, comprenant :
- au moins deux vis (1a ; 1b) ayant chacune un premier tronçon fileté (1a.1 ; 1b.1) pour être engagé dans une vertèbre et un deuxième tronçon fileté (1a.2 ; 1b.2) séparé du premier tronçon fileté par une collerette (2) ;
- au moins une première tige de liaison ayant deux extrémités opposées pour relier l'un à l'autre les deuxièmes tronçons filetés des vis ; et
- au moins un premier ensemble de fixation et un deuxième ensemble de fixation pour fixer les extrémités de la tige aux vis, chaque ensemble de fixation comprenant au moins une pince(50), engageable sur le deuxième tronçon fileté (1.2) d'une des vis et agencée pour serrer une extrémité de la tige, et un écrou (80) qui a une surface inférieure d'appui et qui est agencé pour être vissé sur le deuxième tronçon fileté (1.2) en serrant la pince contre une surface supérieure de la collerette de la vis, chaque ensemble de fixation étant agencé pour créer une liaison rotule entre la pince et le deuxième tronçon fileté.

2. Dispositif selon la revendication 1, dans lequel la surface inférieure d'appui de l'écrou (80) et la surface supérieure d'appui de la collerette (2) sont des surfaces concaves en calotte sphérique identiques l'une à l'autre.

3. Dispositif selon la revendication 1 ou 2, dans lequel la pince (50) au moins du premier ensemble de fixation (F1) comprend un premier mors (51) ayant une surface extérieure convexe en calotte sphérique complémentaire (51.4) pour prendre appui sur la surface supérieure de la collerette (2) et un deuxième mors (52) ayant une surface extérieure convexe (52.4) en calotte sphérique, le premier ensemble de fixation (F1) comprenant une première douille d'appui (60) de l'écrou (80) ayant une surface frontale inférieure complémentaire pour prendre appui contre la surface extérieure convexe en calotte sphérique du deuxième mors.

4. Dispositif selon la revendication 3, dans lequel la première douille d'appui (60) est distincte de l'écrou (80).

5. Dispositif selon la revendication 4, dans lequel la première douille d'appui (60) comprend une surface frontale supérieure convexe en calotte sphérique complémentaire de la surface inférieure de l'écrou pour prendre appui contre celle-ci.

6. Dispositif selon la revendication 1 ou 2, dans lequel la pince (50.2) au moins du premier ensemble de fixation (F2) comprend un premier mors (51) ayant une surface extérieure convexe en calotte sphérique complémentaire pour prendre appui contre la surface inférieure de l'écrou (80) et un troisième mors (53) ayant une surface extérieure concave en calotte sphérique, le premier ensemble de fixation comprenant une deuxième douille (70) ayant une surface frontale supérieure (70.2) complémentaire pour prendre appui sur la surface extérieure concave en calotte sphérique du troisième mors.

7. Dispositif selon la revendication 6, dans lequel la deuxième douille (70) a une surface frontale inférieure concave (70.1) en calotte sphérique complémentaire pour prendre appui sur la surface supérieure de la collerette (2).

8. Dispositif selon l'une quelconque des revendications précédente, dans lequel le deuxième ensemble (F3) comprend deux pinces à savoir une pince inférieure (50.1) et une pince supérieure (50.2), la pince inférieure (50.1) comprenant un premier mors (51) ayant une surface extérieure convexe en calotte sphérique complémentaire (51.4) pour prendre appui sur la surface supérieure de la collerette (2) et un deuxième mors (52) ayant une surface extérieure convexe en calotte sphérique, la pince supérieure (50.2) comprenant un premier mors (51) ayant une surface extérieure convexe en calotte sphérique complémentaire (51.4) pour prendre appui contre la surface inférieure de l'écrou (80) et un troisième mors (53) ayant une surface extérieure concave en calotte sphérique complémentaire (53.4) de la surface extérieure convexe en calotte sphérique (52.4) du deuxième mors (52) pour prendre appui sur celle-ci.

9. Dispositif selon l'une quelconque des revendications 3 à 8, dans lequel chaque pince comprend une portion élastiquement déformable (55) reliant les mors (51, 52 ; 51, 53) de telle manière que ceux-ci soient mobiles l'un par rapport à l'autre entre une position ouverte et une position fermée.

10. Dispositif selon l'une quelconque des revendications 3 à 9, dans lequel le premier mors (51) est pourvu d'un perçage pour être engagé sur le deuxième tronçon fileté (1.2) de la vis (1), le perçage comprenant un premier tronçon tronconique et un deuxième tronçon cylindrique, le premier tronçon ayant une grande section débouchant sur la surface extérieure (51.4) du premier mors (51) et une petite section confondue avec une section cylindrique du deuxième tronçon, le deuxième tronçon débouchant sur la surface intérieure (51.3) du premier mors (51).

11. Dispositif selon les revendications 3 à 10, dans lequel le deuxième mors (52) est pourvu d'un perçage pour être engagé sur le deuxième tronçon fileté (1.2) de la vis (1), le perçage comprenant un premier tronçon tronconique et un deuxième tronçon tronconique, le premier tronçon ayant une grande section débouchant sur la surface extérieure (52.4) du deuxième mors (52) et une petite section confondue avec une petite section du deuxième tronçon, le deuxième tronçon ayant une grande section débouchant sur la surface intérieur (52.3) du deuxième mors (52), le centre commun de rotation étant disposé au contre des petites sections des premier et deuxième tronçons.

12. Dispositif selon l'une quelconque des revendications précédentes, dans lequel la collerette (2) est amovible.

13. Dispositif selon l'une quelconque des revendications précédentes, dans lequel la tige (3) présente sur toute sa longueur une section constante.

14. Dispositif selon l'une quelconque des revendications précédentes, dans lequel la tige (3) est incurvée.

15. Dispositif selon l'une quelconque des revendications précédentes, dans lequel le deuxième tronçon fileté (1.2) de chaque vis (1) est pourvu d'une portion terminale agencée pour autoriser une liaison en rotation avec un outil de vissage de la vis dans la vertèbre et une amorce de rupture pour pouvoir séparer par rupture la portion terminale du deuxième tronçon fileté.

## Patentansprüche

1. Vorrichtung zur Wirbelsäulenstabilisierung, umfassend:
- zumindest zwei Schrauben (1a ; 1b) mit jeweils einem ersten Gewindeabschnitt (1a.1 ; 1b.1), der in einen Wirbel eingedreht wird, und einem zweiten Gewindeabschnitt (1a.2 ; 1b.2), der von dem ersten Gewindeabschnitt durch einen Kragen (2) getrennt ist;
- zumindest einen ersten Verbindungsstab mit zwei entgegengesetzten Enden, um die zweiten Gewindeabschnitte der Schrauben miteinander zu verbinden; und
- zumindest eine erste Befestigungsanordnung und eine zweite Befestigungsanordnung, um die Stabenden an den Schrauben zu befestigen, wobei jede Befestigungsanordnung umfasst: zumindest eine Zange (50), die auf den zweiten Gewindeabschnitt (1.2) einer der Schrauben aufschiebbar ist und dafür ausgelegt ist, ein Stabende einzuspannen, sowie eine Mutter (80), die eine untere Auflagefläche aufweist und dafür ausgelegt ist, auf den zweiten Gewindeabschnitt (1.2) aufgeschraubt zu werden und so die Zange gegen eine obere Fläche des Schraubenkragens festzuspannen, wobei jede Befestigungsanordnung dafür ausgelegt ist, zwischen der Zange und dem zweiten Gewindeabschnitt eine Kugelgelenkverbindung zu bilden.

2. Vorrichtung nach Anspruch 1, wobei es sich bei der unteren Auflagefläche der Mutter (80) und der oberen Auflagefläche des Kragens (2) um konkave Flächen in Form einer Kugelkalotte handelt, die zueinander identisch ausgebildet sind.

3. Vorrichtung nach Anspruch 1 oder 2, wobei die Zange (50) von zumindest der ersten Befestigungsanordnung (F1) eine erste Backe (51) mit einer konvexen Außenfläche in Form einer komplementär ausgebildeten Kugelkalotte (51.4) umfasst, um sich auf der oberen Fläche des Kragens (2) abzustützen, sowie eine zweite Backe (52) mit einer konvexen Außenfläche (52.4) in Form einer Kugelkalotte umfasst, wobei die erste Befestigungsanordnung (F1) eine erste Stützhülse (60) zur Stützung der Mutter (80) umfasst, welche eine komplementär ausgebildete untere Stirnfläche aufweist, um sich gegen die in Form einer Kugelkalotte ausgebildete konvexe Außenfläche der zweiten Backe abzustützen.

4. Vorrichtung nach Anspruch 3, wobei die erste Stützhülse (60) nicht Bestandteil der Mutter (80) ist.

5. Vorrichtung nach Anspruch 4, wobei die erste Stützhülse (60) eine konvex ausgebildete obere Stirnfläche in Form einer Kugelkalotte umfasst, die komplementär zu der unteren Fläche der Mutter ausgebildet ist, um sich gegen diese abzustützen.

6. Vorrichtung nach Anspruch 1 oder 2, wobei die Zange (50.2) von zumindest der ersten Befestigungsanordnung (F2) eine erste Backe (51) mit einer konvexen Außenfläche in Form einer komplementär ausgebildeten Kugelkalotte umfasst, um sich gegen die untere Fläche der Mutter (80) abzustützen, sowie eine dritte Backe (53) mit einer konkaven Außenfläche in Form einer Kugelkalotte umfasst, wobei die erste Befestigungsanordnung eine zweite Hülse (70) umfasst, die eine komplementär ausgebildete obere Stirnfläche (70.2) aufweist, um sich auf der in Form einer Kugelkalotte ausgebildeten konkaven Außenfläche der dritten Backe abzustützen.

7. Vorrichtung nach Anspruch 6, wobei die zweite Hülse (70) eine konkave untere Stirnfläche (70.1) in Form einer komplementär ausgebildeten Kugelkalotte aufweist, um sich auf der oberen Fläche des Kragens (2) abzustützen.

8. Vorrichtung nach einem der vorhergehenden Ansprüche, wobei die zweite Anordnung (F3) zwei Zangen, nämlich eine untere Zange (50.1) und eine obere Zange (50.2), umfasst, wobei die untere Zange (50.1) eine erste Backe (51) mit einer konvexen Außenfläche in Form einer komplementär ausgebildeten Kugelkalotte (51.4) umfasst, um sich auf der oberen Fläche des Kragens (2) abzustützen, sowie eine zweite Backe (52) mit einer konvex ausgebildeten Außenfläche in Form einer Kugelkalotte umfasst, wobei die obere Zange (50.2) eine erste Backe (51) mit einer konvexen Außenfläche in Form einer komplementär ausgebildeten Kugelkalotte (51.4) umfasst, um sich gegen die untere Fläche der Mutter (80) abzustützen, sowie eine dritte Backe (53) mit einer konkaven Außenfläche in Form einer Kugelkalotte (53.4) umfasst, die komplementär zu der konvexen Außenfläche in Form einer Kugelkalotte (52.4) der zweiten Backe (52) ausgebildet ist, um sich auf dieser abzustützen.

9. Vorrichtung nach einem der Ansprüche 3 bis 8, wobei jede Zange einen elastisch verformbaren Abschnitt (55) umfasst, welcher die Backen (51, 52 ; 51, 53) derart miteinander verbindet, dass diese zwischen einer offenen Stellung und einer geschlossenen Stellung in Bezug aufeinander beweglich sind.

10. Vorrichtung nach einem der Ansprüche 3 bis 9, wobei die erste Backe (51) mit einer Bohrung versehen ist, um auf den zweiten Gewindeabschnitt (1.2) der Schraube (1) aufgeschoben zu werden, wobei die Bohrung einen ersten kegelstumpfförmigen Abschnitt und einen zweiten zylindrischen Abschnitt umfasst, wobei der erste Abschnitt einen großen Querschnitt aufweist, der in die Außenfläche (51.4) der ersten Backe (51) mündet, und einen kleinen Querschnitt aufweist, der mit einem zylindrischen Querschnitt des zweiten Abschnitts zusammenfällt, wobei der zweite Abschnitt in die Innenfläche (51.3) der ersten Backe (51) mündet.

11. Vorrichtung nach den Ansprüchen 3 bis 10, wobei die zweite Backe (52) mit einer Bohrung versehen ist, um auf den zweiten Gewindeabschnitt (1.2) der Schraube (1) aufgeschoben zu werden, wobei die Bohrung einen ersten kegelstumpfförmigen Abschnitt und einen zweiten kegelstumpfförmigen Abschnitt umfasst, wobei der erste Abschnitt einen großen Querschnitt aufweist, der in die Außenfläche (52.4) der zweiten Backe (52) mündet, und einen kleinen Querschnitt aufweist, der mit einem kleinen Querschnitt des zweiten Abschnitts zusammenfällt, wobei der zweite Abschnitt einen großen Querschnitt aufweist, der in die Innenfläche (52.3) der zweiten Backe (52) mündet, wobei der gemeinsame Drehpunkt im Zentrum der kleinen Querschnitte des ersten und des zweiten Abschnitts angeordnet ist.

12. Vorrichtung nach einem der vorhergehenden Ansprüche, wobei der Kragen (2) abnehmbar ist.

13. Vorrichtung nach einem der vorhergehenden Ansprüche, wobei der Stab (3) über seine gesamte Länge einen konstanten Querschnitt aufweist.

14. Vorrichtung nach einem der vorhergehenden Ansprüche, wobei der Stab (3) gekrümmt ist.

15. Vorrichtung nach einem der vorhergehenden Ansprüche, wobei der zweite Gewindeabschnitt (1.2) jeder Schraube (1) mit einem Endbereich versehen ist, der dafür ausgelegt ist, eine drehfeste Verbindung mit einem Eindrehwerkzeug zum Eindrehen der Schraube in den Wirbel zu erlauben, sowie mit einer Sollbruchstelle versehen ist, damit der Endbereich des zweiten Gewindeabschnitts durch Abbrechen abgetrennt werden kann.

## Claims

1. Vertebral stabilization device, comprising:
- at least two screws (1a; 1b), each having a first threaded portion (1a.1; 1b.1) to be engaged in a vertebra and a second threaded portion (1a.2; 1b.2) separated from the first threaded portion by a flange (2);
- at least one first connection rod having two opposite ends to connect the two threaded portions of the screws to one another; and
- at least one first securing assembly and one second securing assembly to secure the ends of the rod to the screws, each securing assembly comprising at least one clip (50), which is able to be engaged on the second threaded portion (1.2) of one of the screws and arranged to clamp an end of the rod, and a nut (80) which has a lower bearing surface and which is designed to be screwed on the second threaded portion (1.2) by clamping the clip against an upper surface of the flange of the screw, each securing assembly being arranged to create a ball-and-socket joint connection between the clip and the second threaded portion.

2. Device according to claim 1, wherein the lower bearing surface of the nut (80) and the upper bearing surface of the flange (2) are spherical cap-shaped concave surfaces which are identical to one another.

3. Device according to claim 1 or 2, wherein the clip (50) at least of the first securing assembly (F1) comprises a first jaw (51) having a complementary spherical cap-shaped convex outer surface (51.4) to bear on the upper surface of the flange (2), and a second jaw (52) having a spherical cap-shaped convex outer surface (52.4), the first securing assembly (F1) comprising a first support sleeve (60) of the nut (80) having a complementary lower front surface to bear against the spherical cap-shaped convex outer surface of the second jaw.

4. Device according to claim 3, wherein the first support sleeve (60) is distinct from the nut (80).

5. Device according to claim 4, wherein the first support sleeve (60) comprises a spherical cap-shaped convex upper front surface complementary of the lower surface of the nut to bear against it.

6. Device according to claim 1 or 2, wherein the clip (50.2) at least of the first securing assembly (F2) comprises a first jaw (51) having a complementary spherical cap-shaped convex outer surface to bear against the lower surface of the nut (80) and a third jaw (53) having a spherical cap-shaped concave outer surface, the first securing assembly comprising a second support sleeve (70) having a complementary upper front surface (70.2) to bear on the spherical cap-shaped concave outer surface of the third jaw.

7. Device according to claim 6, wherein the second support sleeve (70) has a complementary spherical cap-shaped concave lower front surface (70.1) to bear on the upper surface of the flange (2).

8. Device according to any one of the preceding claims, wherein the second assembly (F3) comprises two clips, namely a lower clip (50.1) and an upper clip (50.2), the lower clip (50.1) comprising a first jaw (51) having a complementary spherical cap-shaped convex outer surface (51.4) to bear on the upper surface of the flange (2), and a second jaw (52) having a spherical cap-shaped convex outer surface, the upper clip (50.2) comprising a first jaw (51) having a complementary spherical cap-shaped convex outer surface (51.4) to bear against the lower surface of the nut (80) and a third jaw (53) having a spherical cap-shaped concave outer surface (53.4) complementary of the spherical cap-shaped convex outer surface (52.4) of the second jaw (52) to bear on it.

9. Device according to any one of claims 3 to 8, wherein each clip comprises an elastically deformable portion (55) connecting the jaws (51, 52; 51, 53), such that these are movable against one another between an open position and a closed position.

10. Device according to any one of claims 3 to 9, wherein the first jaw (51) is provided with a bore to be engaged on the second threaded portion (1.2) of the screw (1), the bore comprising a first truncated cone-shaped portion and a second cylindrical portion, the first portion having a large cross-section opening onto the outer surface (51.4) of the first jaw (51) and a small cross-section combined with a cylindrical cross-section of the second portion, the second portion opening onto the inner surface (51.3) of the first jaw (51).

11. Device according to claims 3 to 10, wherein the second jaw (52) is provided with a bore to be engaged on the second threaded portion (1.2) of the screw (1), the bore comprising a first truncated cone-shaped portion and a second truncated cone-shaped portion, the first portion having a large cross-section opening onto the outer surface (52.4) of the second jaw (52) and a small cross-section combined with a small cross-section of the second portion, the second portion having a large cross-section opening onto the inner surface (52.3) of the second jaw (52), the common centre of rotation being disposed against the small cross-sections of the first and second portions.

12. Device according to any one of the preceding claims, wherein the flange (2) is removable.

13. Device according to any one of the preceding claims, wherein the rod (3) has a constant cross-section over its entire length.

14. Device according to any one of the preceding claims, wherein the rod (3) is curved.

15. Device according to any one of the preceding claims, wherein the second threaded portion (1.2) of each screw (1) is provided with an end portion designed to enable a rotating connection with a tool for screwing the screw into the vertebra, and a breaking piece to be able to separate the end portion from the second threaded portion by breaking.
